# FASCICULE DE BREVET EUROPEEN

(11) **EP 3 698 366 B1**
(45) Date de publication et mention de la délivrance du brevet: **19.03.2025**
(21) Numéro de dépôt: 18785973.1
(22) Date de dépôt: 18.10.2018
(51) Int. Cl.: G16B 20/00

(54) **PROCÉDÉ ET SYSTÈME DE GÉNÉRATION D'UN IDENTIFIANT UNIQUE D'UN SUJET À PARTIR DE SON ADN**
VERFAHREN UND SYSTEM ZUR ERZEUGUNG EINES EINDEUTIGEN IDENTIFIKATORS EINES PROBANDEN AUS DER DNA DES PROBANDEN
METHOD AND SYSTEM FOR GENERATING A UNIQUE IDENTIFIER OF A SUBJECT FROM THE DNA OF SAID SUBJECT

(30) Priorité: 18.10.2017 FR 1759779
(43) Date de publication de la demande: 26.08.2020
(73) Titulaire: Centre National de la Recherche Scientifique (CNRS), 75016 Paris (FR); Institut National de la Santé et de la Recherche Médicale (INSERM), 75013 Paris (FR); Université Paul Sabatier Toulouse III, 31400 Toulouse (FR)
(72) Inventeur: PIERRON, Denis, 31400 Toulouse (FR); LETELLIER, Thierry, 33110 Le Bouscat (FR); HEISKE, Margit, 31400 Toulouse (FR); PEREDA, Veronica, 31120 Portet sur Garonne (FR)
(74) Mandataire: Novagraaf Technologies
(86) Numéro de dépôt international: PCT/EP2018/078517
(87) Numéro de publication internationale: WO 2019/077025

(56) Documents cités:
- WO-A1-01/06454
- RICKE DARRELL O: "FastID: Extremely fast forensic DNA comparisons", 2017 IEEE HIGH PERFORMANCE EXTREME COMPUTING CONFERENCE (HPEC), IEEE, 12 September 2017 (2017-09-12), pages 1 - 4, XP033244343, DOI: 10.1109/HPEC.2017.8091056
- ZHAOSHU ZENG ET AL: "Evaluation of 96 SNPs in 14 Populations for Worldwide Individual Identification* : EVALUATION OF 96 SNPS IN 14 POPULATIONS", JOURNAL OF FORENSIC SCIENCES, vol. 57, no. 4, 5 March 2012 (2012-03-05), CHICAGO, IL, US, pages 1031 - 1035, XP055493618, ISSN: 0022-1198, DOI: 10.1111/j.1556-4029.2012.02110.x
- EMILIANO GIARDINA ET AL: "In silico and in vitro comparative analysis to select, validate and test SNPs for human identification", BMC GENOMICS, vol. 8, no. 1, 1 January 2007 (2007-01-01), pages 457, XP055493621, ISSN: 1471-2164, DOI: 10.1186/1471-2164-8-457
- LARUE BOBBY L ET AL: "Characterization of 114 insertion/deletion (INDEL) polymorphisms, and selection for a global INDEL panel for human identification", LEGAL MEDICNE, JAPANESE SOCIETY OF LEGAL MEDICINE, TOKYO, JP, vol. 16, no. 1, 1 November 2013 (2013-11-01), pages 26 - 32, XP028670478, ISSN: 1344-6223, DOI: 10.1016/J.LEGALMED.2013.10.006

## Description

### DOMAINE TECHNIQUE GÉNÉRAL

L'invention concerne le domaine de l'authentification biométrique et concerne plus particulièrement la génération d'un identifiant unique d'un sujet à partir de son ADN.

### ETAT DE LA TECHNIQUE

Les enjeux liés à la notion d'identité sont importants et couvrent des domaines très larges qui vont des besoins administratifs (nationalité, droit de vote, santé, retraite, etc.) aux nécessités de sécurité nationale (frontière, criminalité, victimes, disparus), en passant par la protection des biens et des données (banque, informations médicales, etc.). Ces enjeux impliquent une garantie quant à l'identité des individus.

L'utilisation des traits biométriques des individus présente donc un intérêt.

Pour authentifier des individus, plusieurs méthodes basées sur un ou plusieurs traits biométriques ont été mises en oeuvre.

En Inde, par exemple un système d'identification de la population a été mis en place et permet d'attribuer un numéro d'identification national à chaque individu en fonction de ses empreintes digitales ou bien d'une photographie de son iris.

On connait aussi des systèmes de vote utilisant la reconnaissance des empreintes digitales pour identifier les votants.

ZHAOSHUZENG ET AL: "Evaluation of 96 SNPs in 14 Populations for Worldwide Individual Identification* décrit un procédé de génération d'un identifiant à partir d'un échantillon d'ADN dont on sélectionne ceux dont la fréquence allélique est supérieure ou égale à 30% sur l'allèle minoritaire) et décrit qu'il convient d'être supérieur ou égal à 35%.

L'utilisation de ces traits biométriques n'est toutefois pas sans problèmes :
- L'identification par ces traits n'est plus possible dès lors que le trait biométrique observé est altéré (vieillissement, chirurgie esthétique, accident)
- Ces traits sont souvent modifiés durant l'enfance, ainsi il est difficile d'identifier un adolescent/adulte à partir de traits mesurés à sa naissance ou petite enfance.
- Les mesures de ces traits peuvent ne pas être complètement fiables ;
- Le détenteur du trait biométrique peut être mis en danger en cas de tentative d'usurpation du trait biométrique ;
- Il n'est pas possible de changer d'identifiant.

### PRÉSENTATION DE L'INVENTION

L'invention propose de pallier au moins un de des inconvénients en proposant un moyen d'identifier des individus de manière performante et unique tout en garantissant aussi bien la sécurité que la vie privée des individus.

À cet effet, l'invention propose un procédé de génération d'un identifiant unique d'un sujet à partir d'un échantillon d'ADN génotypé dudit sujet, le procédé comprenant les étapes de :
- obtention d'un nombre de positions génomiques d'intérêt, lesdites positions génomiques obtenues étant telles que sur une population de M sujets, ces positions génomiques présentent un polymorphisme binaire ayant une fréquence allélique de 29% ±7% pour l'allèle minoritaire ;
- combinaison deux à deux des positions génomiques obtenues au moyen de fonctions logiques de manière à obtenir un code binaire cb.

L'invention est avantageusement complétée par les caractéristiques suivantes, prises seules ou en une quelconque de leur combinaison techniquement possible :
- la fréquence allélique est de 29 % ;
- le polymorphisme comprend un seul nucléotide (SNP) ;
- les positions combinées deux à deux ne sont pas liées et sont préférentiellement distantes d'au moins 100 centimorgan ou sur des chromosomes différents ;
- le polymorphisme est une insertion ou une délétion d'au moins deux nucléotides ;
- la combinaison consiste à comparer les positions génomiques d'intérêt de la manière suivante : les positions obtenues sont Pi, i=1 à N ; on affecte des états logiques '1' ou '0' comme suit : Pi='1' si sur les deux variations SNPi au moins un allèle 1 est présent, sinon Pi='0' ; combinaison logique deux à deux des Pi pour obtenir le code binaire cb ;
- les positions obtenues sont non codantes pour le sujet et à tout le moins ne comprennent pas d'informations sur le sexe dudit sujet ;
- le procédé comprend une étape de cryptage du code binaire obtenu, le code crypté étant aussi binaire ;
- le procédé comprend une étape de conversion du code binaire obtenu en un code hexadécimal ou en QR code.

L'invention concerne un système de génération d'un identifiant unique d'un sujet à partir d'un échantillon d'ADN génotypé dudit sujet, comprenant un processeur configuré pour mettre en œuvre un procédé selon l'invention.

Les avantages de l'invention sont multiples.

L'invention est fondée sur l'ADN qui est disponible de manière identique dans n'importe quelle partie du corps humain (aux mutations somatiques près).

L'identifiant généré est : unique pour chaque individu, anonyme, stable dans le temps, en particulier il ne se détériore pas et est infalsifiable. En outre, le stockage de l'identifiant est simple car il ne nécessite pas beaucoup d'espace à l'opposé d'un stockage brut des informations génétiques d'un individu.

L'invention permet donc de générer un identifiant unique pour chaque sujet (à l'exception des vrais jumeaux ou clones). Cet identifiant se base sur la diversité génétique de l'espèce étudiée à travers le monde, la connaissance de cette diversité permet de choisir spécifiquement des positions génomiques et de calculer mathématiquement la probabilité que des sujets possèdent le même identifiant. Il est ainsi possible de démontrer qu'une centaine de positions adéquatement choisies vont suffire à rendre nulle cette probabilité. La méthode proposée ici repose sur la notion de couple de position. En effet en utilisant deux positions (soit un couple de position), la probabilité que deux sujets pris au hasard partagent le même code est ½. La formule générale correspondante est 0,5ⁿ avec n le nombre de couple de positions étudiées. Ainsi 100 positions étudiées correspondent à 50 couples, soit une probabilité de 0,5⁵⁰ ou 10⁻¹⁵.

L'identifiant en soit est anonyme en ce qu'il est impossible d'obtenir aucune information sur la personne (âge, lieu de naissance, sexe, région d'origine, caractéristiques physiques, caractéristiques génomique). Autrement dit, l'identifiant d'une personne ne donne aucune information qui pourrait potentiellement aider à le caractériser d'une quelconque façon. Du fait de sa conception et des caractéristiques des marqueurs génétiques, il est impossible d'obtenir d'informations du génome utilisé à partir de l'identifiant, cela même pour les détenteurs de l'algorithme ayant été utilisé pour générer l'identifiant.

L'invention permet d'obtenir un identifiant foncièrement différent entre deux sujets, qu'ils soient pris au hasard ou qu'ils appartiennent à la même famille. Le fait d'appartenir à une même population n'a aucune influence sur l'identifiant. Par exemple, pour deux identifiants composés de 20 hexadécimaux, la probabilité que deux sujets partagent le même identifiant est inférieure à 1 chance sur 72 millions de milliards.

L'analyse ADN d'un individu donnera toujours le même identifiant infalsifiable et intemporel. L'identifiant est donc intemporel. Ainsi, de sa naissance à sa mort, un individu aura toujours le même génome (et de facto le même identifiant). L'ADN se conservant bien post-mortem il est possible de vérifier l'identifiant d'un individu longtemps après sa mort (il est même possible de générer l'identifiant d'un individu mort depuis 40 000 ans).

Avantageusement, l'identifiant final est un code hexadécimal qui peut se lire facilement et qui permet une identification instantanée avec des moyens informatiques en le comparant à un registre comptant plusieurs millions ou même milliards de numéros.

Les applications de l'invention sont multiples.

Il est possible d'utiliser la même méthodologie exposée ici pour créer différents types d'identifiants (un identifiant spécifique aux applications juridiques, un autre pour les applications bancaires, etc.) pour un sujet (en changeant par exemple les positions choisies).

L'identifiant permet de répondre aux problèmes d'authentification et de secret bancaire, l'invention permettant de générer des identifiants indépendants pour chaque application.

Cet identifiant peut permettre à la police scientifique d'identifier un individu.

Cet identifiant permet, par exemple, d'ouvrir un compte en banque. Ainsi seule la personne dont le génome correspond à l'identifiant peut accéder au contenu du compte.

Cet identifiant permet, par exemple, d'ouvrir un compte pour y stocker des papiers personnels (propriétés, identifications) ou de l'argent pour soi-même ou ses enfants avec une garantie que le bénéficiaire puisse y accéder dans le futur. Ceci est particulièrement utile en cas d'événement catastrophique lors de la perte de papiers d'identification ou autre moyen habituel d'identification (inondation, feux, guerre, décès soudain de deux parents, etc...).

### PRÉSENTATION DES FIGURES

D'autres caractéristiques, buts et avantages de l'invention ressortiront de la description qui suit, qui est purement illustrative et non limitative, et qui doit être lue en regard des dessins annexés sur lesquels :
- la figure 1 illustre un système de génération d'un identifiant selon un mode de réalisation de l'invention ;
- la figure 2 illustre un procédé de génération d'un identifiant selon un mode de réalisation de l'invention ;
- La figure 3 illustre des données sur des séquences mises en œuvre dans l'invention.

### DESCRIPTION DÉTAILLÉE DE L'INVENTION

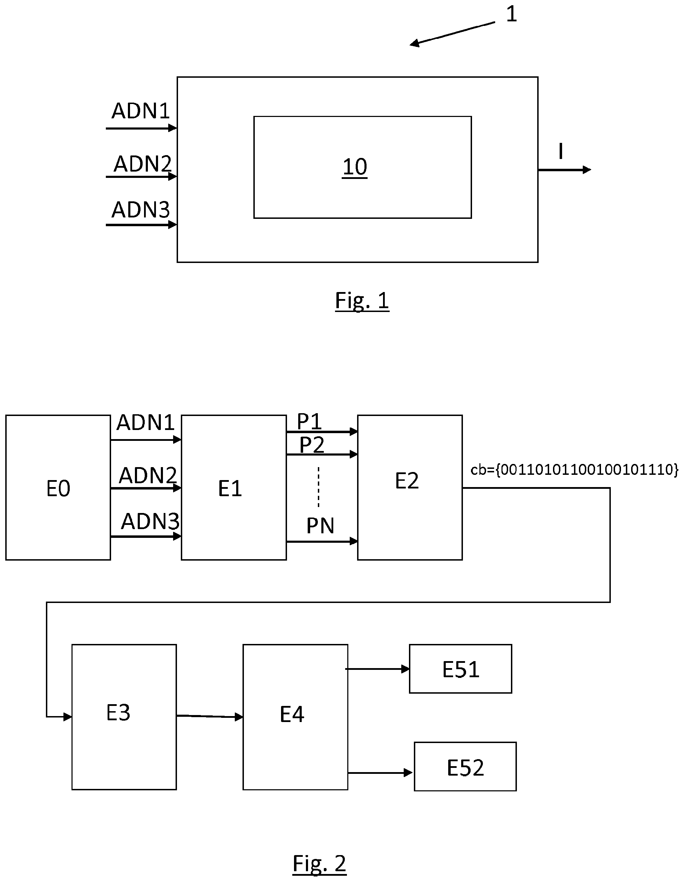

En relation avec la **figure** 1 un système 1 de génération d'un identifiant d'un sujet à partir d'un échantillon d'ADN dudit sujet comprend une unité 10 de traitement (par exemple un processeur) configurée pour mettre en œuvre un procédé de génération d'un identifiant à partir d'un échantillon d'ADN décrit ci-après en relation avec la **figure 2****.**

Le sujet ou l'individu peut être toute entité composée d'ADN (un être humain, un animal, une plante, etc.).

Dans une étape préliminaire (étape E0), au moins un échantillon d'ADN ADN1, ADN2, ADN3 est prélevé sur un sujet. De préférence, des échantillons sont prélevés à plusieurs endroits différents (trois endroits différents par exemple). L'échantillon est par exemple de la salive, du sang, des cheveux, etc. ou de manière plus générale tout ce qui permet de prélever de nombreux types d'échantillons biologiques contenant des cellules de l'individu.

A partir des échantillons prélevés, l'ADN du sujet est séquencé/génotypé (étape E1).

Le séquençage/génotypage permet d'obtenir des positions génomiques d'intérêt (étape E2) Pi, i=1, ..., N.

Le génotypage peut cibler ou non les positions d'intérêt et peut être généré par de multiples procédures (puces, séquençage ou autre). Ainsi le procédé ici décrit est utilisable aussi bien à partir de résultats de puces générées à façon qu'à partir d'un séquençage complet du génome de l'individu (seules seront gardées les positions d'intérêts). Le nombre de positions utilisées est dépendant du niveau d'unicité et de sécurité voulu suivant la formule présentée plus haut. En d'autres termes les étapes E1 et E2 peuvent être implémentées simultanément.

Le nombre total de positions étudiés peut typiquement être de 100.

Les positions génomiques d'intérêt ne sont pas arbitraires mais sont telles qu'en considérant M sujets (le sujet pour lequel on génère le code peut ou ne pas appartenir à ces M sujets) ces positions présentent un polymorphisme binaire ayant une fréquence allélique de 29% ±7% dans la population. En outre ces positions respectent l'équilibre de Hardy Weinberg et la position n'est pas située sur un chromosome sexuel ou sur l'ADNmt.

Plus précisément, la fréquence allélique idéale du polymorphisme est de 29%.

Le polymorphisme considéré sur les M sujets peut, de façon la plus simple, ne concerner qu'un seul nucléotide (SNP en anglais pour *"Single Nucleotide Polymorphism*")*.* De manière alternative, le polymorphisme considéré sur les M sujets peut concerner deux nucléotides mais il peut s'agir aussi d'insertion ou de délétion de nucléotide (INDEL).

Bien entendu, le polymorphisme le plus simple est celui pris sur un seul nucléotide.

Le polymorphisme est binaire du fait que les différences considérées ne sont qu'entre deux allèles différents d'une position. On considère donc un allèle présentant un état '1' et un allèle avec un état '0'. Cette convention facilite les étapes ultérieures du procédé.

De préférence, mais non limitativement, les positions étudiées ne sont pas localisées sur une partie codante (gène) du génome.

Nous reviendrons plus loin sur ces séquences.

Les polymorphismes obtenus sont alors combinés entre eux, deux à deux, au moyen de fonctions logiques pour obtenir un code binaire cb (étape E3).

Dans le cas introduit, d'un polymorphisme binaire, la combinaison consiste à comparer les positions de la manière suivante :
- les positions obtenues sont Pi, i=1 à N ;
- on affecte des états logiques '1' ou '0' comme suit : Pi='1' si sur les deux variations du SNPi au moins un allèle 1 est présent, sinon Pi='0'.
- on combine deux à deux des Pi pour obtenir le code binaire cb.

Chaque élément du code binaire correspond à une combinaison de deux séquences.

La combinaison est mise en œuvre au moyen d'une fonction logique.

Une fonction logique est par exemple : OU Exclusif (XOR), Ou (OR), ET (AND), NON-OU (NOR), NON-ET (NAND) ...

Bien entendu, la fonction logique peut être une combinaison plus ou moins complexe de ces fonctions logiques élémentaires.

Alternativement et de manière plus générale, la combinaison peut obéir à tout type de fonction afin d'avoir un maximum de possibilités de combinaisons envisageables.

Indépendamment de la logique combinatoire choisie, il convient d'avoir des combinaisons de positions dont le génotype peut être considéré comme indépendant c'est-à-dire qu'elles ne seront pas en déséquilibre de liaison Pour éviter cette possibilité, il convient de choisir des positions physiquement éloignées l'une de l'autre c'est-à-dire idéalement sur des chromosomes différents ou distantes de plus de 100 centimorgan si elles sont situées sur le même chromosome. (Sachant qu'une distance de 50 centimorgans représente une probabilité de 50% de chance de recombinaison entre deux positions lors d'une génération).

Ce code binaire obtenu, ce dernier peut être crypté (étape E4) mais ce n'est pas obligatoire. Le cryptage du code binaire permet de protéger le code binaire obtenu.

De manière complémentaire, le code binaire crypté ou non est converti en un code hexadécimal (étape E51) ou en un QR code (étape E52). En effet, un code hexadécimal (c'est-à-dire une suite d'une vingtaine de caractères hexadécimaux pouvant être directement traduits du binaire en utilisant une table de référence) est plus facile à stocker dans une mémoire par exemple et à lire. De même, une forme graphique de ce code de type code-barres ou QR code pourra être produit afin de permettre une lecture optique automatisée.

Comme indiqué les positions d'intérêt dans le génome sont celles qui, sur une population de M sujets, présentent un polymorphisme de fréquence allélique de 29% ±7%.

Dans le cas des êtres humains, les sujets sont issus de nombreux pays sur tous les continents, une position ne sera d'intérêt uniquement si la fréquence allélique est homogène et va de 29% ±7% dans toutes les populations. Un polymorphisme qui serait absent dans une population ou bien à 100% dans une autre serait exclu. De la même façon toutes positions associées à une origine ethnique ou géographique de l'individu sont exclues.

Les positions sont dites d'intérêt si les fréquences respectent les prédictions des modèles de Hardy-Weimberg (c'est le cas de la très vaste majorité des polymorphismes). D'après ce modèle, la fréquence mentionnée correspond à des positions dont le polymorphisme est présent chez 50% des sujets.

Concrètement, considérons un locus polymorphique X de l'ADN de fréquence allélique 29% qui possède 2 allèles : 1 et 0, ainsi dans une population, les individus peuvent être homozygotes (11 ou 00) ou bien hétérozygotes (10 et 01) pour ce locus. Nous pouvons classer les individus selon deux états, A ou B :
- l'état A représente le fait d'être homozygote pour l'allèle majoritaire (11)
- l'état B représente le fait d'être homozygote pour l'allèle minoritaire (00) ou bien d'être hétérozygote (10 et 01)

Nous définissons comme "communs" les polymorphismes pour lesquels les 2 états (A et B) sont équiprobables. C'est-à-dire qu'un individu a une même probabilité d'être A ou B (soit 50% chacun).

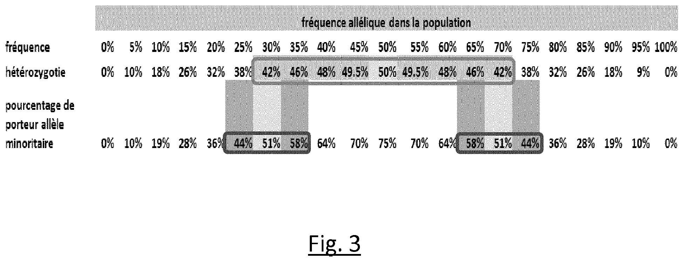

**La** **figure** 3 illustre les relations entre fréquences alléliques, taux d'hétérozygotie et pourcentages de porteur de l'allèle minoritaire. Sur cette figure, plusieurs informations sur les séquences sont reportées (obtenues selon les principes de Hardy-Weimberg) : la première ligne indique la fréquence allélique dans la population de M sujets, la deuxième ligne indique le pourcentage d'hétérozygotie, la troisième ligne indique le pourcentage de porteur de l'allèle minoritaire. On constate que pour des positions ayant une fréquence allélique de 29% ±7%, on a entre 44% et 58% de porteurs de l'allèle considéré (soit environ une personne sur deux) de sorte qu'il y a quasi équiprobabilité que deux individus portent cette séquence. Ainsi, compte tenu du nombre de données, et du fait que les codes sont obtenus au moyen de plusieurs séquences, l'unicité du code pour un sujet est garantie.

Outre ces caractéristiques de fréquence les positions doivent répondre à des critères supplémentaires : comme mentionné précédemment, les positions ne doivent pas être situés sur les chromosomes sexuels ni sur le chromosome mitochondrial. De plus, seront privilégiées des positions qui ne sont pas situées sur des zones codantes du génome et qui ne sont pas associées à des caractéristiques physiques, comportementales ni associées à une prédisposition à certaines maladies. L'association de ces positions est remarquable en ce qu'elle permet d'identifier les sujets sans donner d'informations sur le sujet.

On peut résumer les critères de la manière suivante.

Critère 1 : Ces polymorphismes possèdent une fréquence mondiale des homozygotes porteurs de l'allèle minoritaire de 50 % (±10 %) ce qui équivaut à une fréquence allélique de 29 % (±7 %).

Critère 2 : Pour qu'un individu ait une probabilité identique d'être A ou B, il faut aussi que la fréquence du polymorphisme ne varie pas grandement à travers les populations humaines, c'est pourquoi non seulement la fréquence mondiale de l'allèle minoritaire du polymorphisme doit être de 29% (±7%) mais cette fréquence ne doit pas varier de plus de 10% entre 2 populations.

Critère 3 : Les caractéristiques de l'individu en lui-même (sexe ou autres caractéristiques physiques, etc.) ne peuvent pas être liées à sa probabilité d'être A ou B. Ainsi les polymorphismes communs situés uniquement sur les chromosomes autosomaux (et pas sur les chromosomes sexuels) ne doivent pas être reliés à des phénotypes ou pathologies connues.

A titre d'exemple ci-dessous une liste de SNPs sur le chromosome 14 satisfaisant ces caractéristiques :

| snp | chromosome | position |
|---|---|---|
| rs111063713 | 14 | 104698456 |
| rs2416015 | 14 | 47511528 |
| rs4991272 | 14 | 59973009 |
| rs3985117 | 14 | 47511530 |
| rs7154083 | 14 | 83441172 |
| rs1686571 | 14 | 20279151 |
| rs57101715 | 14 | 90785895 |
| rs55795050 | 14 | 90782024 |
| rs8023212 | 14 | 90777846 |
| rs11845387 | 14 | 90785495 |
| rs574386930 | 14 | 96967163 |
| rs2792111 | 14 | 20418489 |
| rs148942138 | 14 | 79600280 |
| rs3742666 | 14 | 90767860 |
| rs118058626 | 14 | 39256730 |
| rs551716374 | 14 | 44995118 |
| rs4988990 | 14 | 101296722 |
| rs2635566 | 14 | 20418538 |
| rs67963498 | 14 | 41329594 |
| rs4902256 | 14 | 64406065 |

## Revendications

1. Procédé de génération d'un identifiant (I) unique d'un sujet à partir d'un échantillon d'ADN génotypé dudit sujet, le procédé comprenant les étapes de :
- obtention (E2) d'un nombre N≥2 de positions génomiques d'intérêt, lesdites positions génomiques obtenues étant telles que sur une population de M sujets, ces positions génomiques présentent un polymorphisme binaire ayant une fréquence allélique de 29% ±7% pour l'allèle minoritaire ;
- combinaison (E3) deux à deux des positions génomiques obtenues au moyen de fonctions logiques de manière à obtenir un code binaire cb.

2. Procédé selon la revendication 1, dans lequel la fréquence allélique est de 29 %.

3. Procédé selon l'une des revendications précédentes, dans lequel le polymorphisme comprend un seul nucléotide (SNP).

4. Procédé selon l'une des revendications précédentes, dans lequel les positions combinées deux à deux ne sont pas liées et sont préférentiellement distantes d'au moins 100 centimorgan ou sur des chromosomes différents.

5. Procédé selon l'une des revendications précédentes, dans lequel le polymorphisme est une insertion ou une délétion d'au moins deux nucléotides.

6. Procédé selon l'une des revendications précédentes, dans lequel la combinaison consiste à comparer les positions génomiques d'intérêt de la manière suivante :
- les positions obtenues sont Pi, i=1 à N ;
- on affecte des états logiques '1' ou '0' comme suit : Pi='1' si sur les deux variations du SNPi au moins un allèle 1 est présent, sinon Pi='0' ;
- combinaison logique deux à deux des Pi pour obtenir le code binaire cb.

7. Procédé selon l'une des revendications précédentes, dans lequel les positions obtenues sont non codantes pour le sujet et à tout le moins ne comprennent pas d'informations sur le sexe dudit sujet.

8. Procédé selon l'une des revendications précédentes, comprenant une étape (E4) de cryptage du code binaire obtenu, le code crypté étant aussi binaire.

9. Procédé selon l'une des revendications précédentes, comprenant une étape de conversion (E51, E52) du code binaire obtenu en un code hexadécimal ou en QR code.

10. Système de génération d'un identifiant (I) unique d'un sujet à partir d'un échantillon d'ADN génotypé dudit sujet, comprenant un processeur (10) configuré pour mettre en œuvre un procédé selon l'une des revendications précédentes.

## Patentansprüche

1. Verfahren zur Erzeugung eines eindeutigen Identifikators (I) eines Subjekts aus einer genotypisierten DNA-Probe des Subjekts, wobei das Verfahren die folgenden Schritte umfasst:
- Erhalten (E2) einer Anzahl N≥ 2 von genomischen Positionen, die von Interesse sind, wobei die erhaltenen genomischen Positionen so beschaffen sind, dass in einer Population von M Probanden diese genomischen Positionen einen binären Polymorphismus mit einer allelischen Häufigkeit von 29 % ± 7 % für das Minoritätsallel aufweisen;
- die paarweise Kombination (E3) der mittels logischer Funktionen erhaltenen genomischen Positionen, um einen Binärcode bc zu erhalten.

2. Verfahren nach Anspruch 1, wobei die Allelfrequenz 29 % beträgt.

3. Verfahren nach einem der vorhergehenden Ansprüche, wobei der Polymorphismus ein Einzelnukleotid-Polymorphismus (SNP) ist.

4. Verfahren nach einem der vorhergehenden Ansprüche, wobei die paarweise kombinierten Positionen nicht miteinander verbunden sind und vorzugsweise mindestens 100 Zentimorgane voneinander entfernt oder auf verschiedenen Chromosomen liegen.

5. Verfahren nach einem der vorhergehenden Ansprüche, wobei der Polymorphismus eine Insertion oder Deletion von mindestens zwei Nukleotiden ist.

6. Verfahren nach einem der vorhergehenden Ansprüche, wobei die Kombination aus dem Vergleich der interessierenden genomischen Positionen wie folgt besteht:
- die erhaltenen Positionen sind Pi, i = 1 bis N;
- Die logischen Zustände "1" oder "0" werden wie folgt zugeordnet: Pi = '1', wenn auf beiden Varianten des SNPi mindestens ein Allel 1 vorhanden ist, ansonsten Pi = '0';
- logische paarweise Kombination von Pi, um den Binärcode bc zu erhalten.

7. Verfahren nach einem der vorhergehenden Ansprüche, wobei die erhaltenen Positionen für das Subjekt nicht codierend sind und zumindest keine Informationen über das Geschlecht des Subjekts enthalten.

8. Verfahren nach einem der vorhergehenden Ansprüche, umfassend einen Schritt (E4) der Verschlüsselung des erhaltenen Binärcodes, wobei der verschlüsselte Code ebenfalls binär ist.

9. Verfahren nach einem der vorhergehenden Ansprüche, umfassend einen Schritt der Umwandlung (E51, E52) des erhaltenen Binärcodes in einen Hexadezimalcode oder QR-Code.

10. System zur Erzeugung eines eindeutigen Identifikators (I) eines Subjekts aus einer genotypisierten DNA-Probe des Subjekts, mit einem Prozessor (10), der so konfiguriert ist, dass er ein Verfahren nach einem der vorhergehenden Ansprüche implementiert.

## Claims

1. A process for generating a unique identifier (I) of a subject from a genotyped DNA sample of said subject, the process comprising the steps of:
- obtaining (E2) a number N ≥ 2 of genomic positions of interest, said genomic positions obtained being such that, in a population of M subjects, these genomic positions have a binary polymorphism having an allelic frequency of 29% ±7% for the minority allele;
- combining (E3) pairwise the genomic positions obtained by means of logic functions so as to obtain a binary code bc.

2. The process as claimed in claim 1, wherein the allelic frequency is 29%.

3. The process as claimed in one of the preceding claims, wherein the polymorphism is a single-nucleotide polymorphism (SNP).

4. The process as claimed in one of the preceding claims, wherein the positions combined pairwise are not linked and are preferably at least 100 centimorgan apart or on different chromosomes.

5. The process as claimed in one of the preceding claims, wherein polymorphism is an insertion or deletion of at least two nucleotides.

6. The process as claimed in one of the preceding claims, wherein the combination consists of comparing genomic positions of interest as follows:
- the positions obtained are Pi, i = 1 to N;
- logic states '1' or '0' are assigned as follows: Pi = '1' if on both variations of the SNPi at least one allele 1 is present, otherwise Pi = '0';
- logical pairwise combination of Pi to obtain the binary code bc.

7. The process as claimed in one of the preceding claims, wherein the positions obtained are non-coding for the subject and at least do not include information on the sex of said subject.

8. The process as claimed in one of the preceding claims, comprising a step (E4) of encrypting the binary code obtained, the encrypted code also being binary.

9. The process as claimed in one of the preceding claims, comprising a step of converting (E51, E52) the binary code obtained into a hexadecimal code or QR code.

10. A system for generating a unique identifier (I) of a subject from a genotyped DNA sample of said subject, comprising a processor (10) configured to implement a process as claimed in one of the preceding claims.
